Europäisches Patentamt

European Patent Office

Office européen des brevets

(19)

(11) Veröffentlichungsnummer: 0 184 640
A2

# (12) EUROPÄISCHE PATENTANMELDUNG

(21) Anmeldenummer: 85113192.0

(22) Anmeldetag: 17.10.85

(51) Int. Cl.⁴: **C 12 N 5/00**

(30) Priorität: 07.11.84 DE 3440557

(43) Veröffentlichungstag der Anmeldung: 18.06.86 Patentblatt 86/25

(84) Benannte Vertragsstaaten: **AT BE CH FR GB IT LI LU NL SE**

(71) Anmelder: **Siegel, Rolf, Dr., Waidmannsteige 1, D-8700 Würzburg 25 (DE)**

(72) Erfinder: **Dähn-Siegel, Sabine, Waidmannsteige 1, D-8700 Würzburg (DE)**

(74) Vertreter: **von Füner, Alexander, Dr. et al, Patentanwälte Schiff, von Füner Strehl, Schübel-Hopf, Ebbinghaus, Finck Mariahilfplatz 2 u. 3, D-8000 München 90 (DE)**

(54) **Unterlage für das Zellwachstum in vitro und deren Verwendung.**

(57) Gegenstand der Erfindung ist eine Unterlage für das Zellwachstum in vitro, dadurch herstellbar, daß man synthetische oder natürliche Lipoide als Reinsubstanzen oder als mengenmäßig definierte Mischungen in einem unpolaren Lösungsmittel oder -gemisch löst oder suspendiert, die erhaltene Lösung oder Suspension mit einem Polyamid, Polypropylen, Polyalkylenterephthalat oder Fluorpolymerisat mit einer Durchlässigkeit von Molekülen mit einem Molekulargewicht von mindestens 400 000 Dalton oder einem Partikeldurchmesser von 500 nm, mechanischer Stabilität und Flexibilität und kurzzeitiger Beständigkeit gegen ein unpolares Lösungsmittel oder -gemisch in innigen Kontakt bringt und anschließend das Lösungsmittel oder -gemisch wieder vollständig entfernt, sowie die Verwendung dieser Unterlage nach Ausbildung eines dichten Zellrasens auf ihrer Oberfläche zum induzierten, stoffwechselaktiven, vektoriellen Stofftransport.

v. FÜNER     EBBINGHAUS     FINCK
PATENTANWÄLTE     EUROPEAN PATENT ATTORNEYS
MARIAHILFPLATZ 2 & 3, MÜNCHEN 90
POSTADRESSE: POSTFACH 95 01 60, D-8000 MÜNCHEN 95

Dr. Rolf Siegel                      EPAA-33152.3

                                      17. Oktober 1985

## UNTERLAGE FÜR DAS ZELLWACHSTUM IN VITRO UND DEREN VERWENDUNG

Bisher können Stoffwechselprodukte von in Zellkultur wachsenden Zellen nur aus dem Nährmedium entnommen werden, da die Zellen auf festen Oberflächen, wie Roux-Flaschen oder "beads" wachsen. Bei dieser Anordnung ist es nicht möglich, daß Stoffwechselprodukte oder andere interessierende Produkte in konzentrierter Form gewonnen werden.

Die im folgenden beschriebene Unterlage soll es ermöglichen, Stoffwechselprodukte in konzentrierter Form zu gewinnen bzw. Stoffwechselleistungen von Zellen in Zellkultur besser auszunutzen. Der Begriff Zellkultur wird hier umfassender als bisher verstanden: auch Anordnungen von normalerweise einzelligen Organismen oder zusammenhängenden Zellverbänden werden hierunter subsumiert.

Diese vorstehend geschilderten Vorteile werden mit der erfindungsgemäßen Unterlage (vgl. Anspruch 1) erzielt.

Erfindungsgemäß werden zunächst als Unterlage verwendet:

a) Polyamid, Polypropylen, Polyalkylenterephthalat oder Fluorpolymerisat;

b) dieses Material hat von seiner Textur her für Moleküle mit einem Molekulargewicht von mindestens 400 000 Dalton oder für einen Partikeldurchmesser von mindestens 500 nm gut durchlässig zu sein und

0184640

c) muß das Material mechanisch stabil und flexibel sein.

Die Beschichtung des die Unterlage bildenden Materials erfolgt dadurch, daß man synthetische und/oder natürliche Lipoide als Reinsubstanzen oder als mengenmäßig definierte Mischungen in einem unpolaren Lösungsmittel(gemisch) löst bzw. suspendiert, diese Lösung bzw. Suspension mit dem Material in innigen Kontakt bringt und das Lösungsmittel(gemisch) anschließend (durch Verdampfen) wieder vollständig entfernt. Als Lösungsmittel kommen unpolare Lösungsmittel wie Chloroform, Hexan, Methylenchlorid und Petroläther sowie hydrophile organische Lösungsmittel wie Methanol, Butanol und Aceton in Frage, ebenso wie Mischungen aus diesen Flüssigkeiten. Erforderlichenfalls können auch Pufferlösungen Bestandteil des zur Lösung oder Suspension benötigten Lösungsmittelgemischs sein.

Bei dieser Vorgehensweise lagern sich die Lipoide idealerweise unter Ausbildung einer monomolekularen Schicht, über van der Waals'sche und hydrophobe Kräfte fest an die Materialoberfläche an, wobei der hydrophile aus Kohlenhydratgruppenketten und/oder Proteinen bestehende Molekülanteil die unmittelbare Trägermaterialoberfläche darstellt und "Ankermöglichkeiten" für die Zellen bietet (siehe auch H. Rauvala, Trends in Biochemistry Science, 7, 323 - 325, 1983).

Bei den synthetischen und/oder natürlichen Lipoiden besteht der hydrophobe Molekülanteil in der Regel aus einer Kohlenwasserstoffkette mit 4 bis ca. 20 C-Atomen, wobei aber deren hydrophober Charakter wichtiger als die chemische Uniformität ist. Natürliche Lipoide können problemlos als Lipoid-Extrakt aus tierischen oder pflanzlichen Materialien gewonnen werden und umfassen je nach Aufarbeitung und Ausgangsmaterial unterschiedliche Mengen an Glycerinphosphatiden, Sphingolipoiden, Glykosphingolipoiden und Lipoproteinen. Wegen des hohen Anteils an (verzweigten) Kohlenhydratgruppenketten hat sich die Gewinnung dieser Substanzen aus Erythrozyten, Synovia, Hirn und Plazenten bewährt. Im einzelnen besteht der hydrophile Anteil der in Reinsubstanzen oder als Mischungen eingesetzten Lipoide aus Kohlenhydraten wie Fucose, Mannose, Galaktose, Glucose, N-Acetylgalactosamin, N-Acylneuraminsäure und Glykosaminglykanen, Verbindungen, von denen bekannt ist, daß sie ein geordnetes Zellwachstum begünstigen. Es besteht auch ohne weiteres die Möglichkeit, beide Seiten der als Membran dienenden Unterlage mit Kohlenhydratgruppenketten und/oder Proteinen unterschiedlicher Zusammensetzung zu beschichten, so daß es eine "zellspezifische" und eine "substratspezifische" Membranseite gibt, was weiter unten noch verdeutlicht wird.

Aus dem oben Ausgeführten ergibt sich, daß als Unterlage Polyamide, Polyäthylen, Polypropylen, Polyalkylenterephthalate und Fluorpolymerisate mit den vorstehend geforderten Eigenschaften der Moleküldurchlässigkeit, Stabilität und Flexibilität eingesetzt werden. Wesentlich für die Eignung zur Beschichtung ist dabei eine relativ kurzzeitige Beständigkeit gegen das verwendete Lösungsmittel(gemisch) mit den darin gelösten Lipoiden.

Beispielsweise kann Polypropylengewebe mit Siebfilter-Textur (10 µm Maschenweite und 100 µm Fadenstärke) eingesetzt werden: das Zellwachstum beginnt auf der Oberfläche der Fäden, im weiteren Verlauf werden dann auch die Filterporen durch die Zellen verschlossen. Die Anzahl der Zellen pro Filteroberfläche liegt dabei deutlich höher als bei der Verwendung von gleichflächigen Roux-Flaschen, da hier ein quasi drei-dimensionales Zellwachstum stattfindet. Weiterhin können auch Filter mit glatten Oberflächen (beispielsweise aus Polycarbonat oder derivatisierter Cellulose, Porenweite zwischen 0,05 und 8 µm beliebig wählbar) verwendet werden: bei diesen Filtern kommt es zu einem gleichmäßig zusammenhängenden Monolayerzellwachstum auf der erfindungsgemäß beschichteten Materialoberfläche.

Das gemäß der obigen Beschreibung beschichtete Material wird nun entweder als Filterkerze ausgebildet (bzw. eine Filterkerze wurde wie beschrieben beschichtet) oder wird in eine geeignete Vorrichtung derart gespannt, daß das beschichtete Material nach Hinzufügen von Nährmedium eine Trennwand darstellt: entweder mit einem inneren und äußeren Kompartiment oder mit zwei voneinander abgetrennten Bereichen.

Das beschichtete Material wird dann auf einer Seite mit Zellen in bekannter Art und Weise beimpft und mit einem geeigneten Nährmedium so in innigen Kontakt gebracht, daß Innen- und Außenseite der Filterkerze bzw. Ober- und Unterseite (linke und rechte Seite) des eingespannten beschichteten Materials vom Nährmedium benetzt sind. Die Zellen läßt man dann unter den üblichen Bedingungen so weit wachsen, bis sich ein zusammenhängender, dichter Zellrasen gebildet hat.

Nun beginnt man die Zusammensetzung des Nährmediums in den Kompartimenten (bzw. auf jeder Seite) kontinuierlich langsam gegensinnig zu ändern (Induktionsphase): beispielsweise erhöht man bei einem auf der Innenseite einer Filterkerze gewachsenen Nierenepithel-Zellrasen die Natrium-Ionenkonzentration im inneren Kompartiment und senkt sie gleichzeitig im äußeren Kompartiment. Die Zellen reagieren hierauf mit einem Ausgleich der Elektrolytkonzentrationen. Bei weiterer kontinuierlicher Erhöhung der intraluminalen Elektrolytkonzentration, bei dann konstanter extraluminaler Elektrolytkonzentration, kommt es durch Aktivierung energieabhängiger Transportsysteme zu einem vektoriell gerichteten Stofftransport: es wird ein nach außen gerichteter Na-Ionen-Konzentrationsgradient aufgebaut und auch aufrechterhalten. (Nutzphase).

Zur Beimpfung der als Membran dienenden Unterlage werden Zellen mit der potentiellen Fähigkeit zum vektoriellen Stofftransport verwendet, wie z.B. Epithelzellen aus Niere, Gastrointestinaltrakt und Drüsengewebe. Ebenfalls geeignet sind Zellen von Schwämmen (Spongien) oder Hohltieren (Coelenterata). Die verwendeten Nährmedien sind auf die Erfordernisse der Zellen abgestimmt und bekannt, ebenso wie die erforderlichen Kulturbedingungen.

Welche Bestandteile des Nährmediums nach Ausbildung des Zellrasens variiert werden, hängt davon ab, welche Stoffe man in konzentrierter Form gewinnen will: für die Konzentration von Zuckern, beispielsweise Galaktose, wird durch gegensinnige Änderung der Galaktose-Konzentration in den zwei Kompartimenten ein vektoriell gerichtetes stoffwechselenegieabhängiges Transportsystem für Galaktose induziert und aufrechterhalten. Analoges gilt für andere transzellulär transportierbare Substanzen. Auf den Aufbau eine $H^+$-Ionen konzentrierenden Systems mit Belegzellen zur Nutzung der bei ca. 400 mV liegenden Potentialdifferenz wird ebenso hingewiesen wie auf die Herabsetzung des Ionengehaltes des Meerwassers mit Hilfe von Zellkulturen aus dem Salzorgan von an Salzwasser adaptierten Süßwasserfischen, mit dem Ziel der biologischen Meerwasserentsalzung.

Beispiel

Als Ausgangsmaterial für das erfindungsgemäße Material wird ein Siebfilter aus Polypropylen (Scheibenfilter mit 60 mm Durchmesser, Porenweite 8 μm, Fadenstärke 100 μm) verwendet.

Die zur Beschichtung des Filters erforderlichen Substanzen werden als Gesamtlipid-Extrakt aus Vollblut - in Anlehnung an Folch et al. (J. Folch, M. Lees and G.E.S. Stanley: J. Biol. Chem. 226, 497, 1957) - gewonnen: ein Teil EDTA-Blut wird mit 9 Teilen eines Chloroform/Methanol-Gemisches extrahiert: die resultierende Chloroform/Methanol-Phase wird mit 0,1 Volumen-Teilen einer mit Ringerlösung gesättigten Acetonlösung versetzt und geschüttelt, die resultierende unter Phase wird weiterverwendet.

Diese Lösung wird nun mit dem Filter in einem geeigneten Behältnis derart in innigen Kontakt gebracht, daß die Lösung ständig den Filter gleichmäßig benetzt und das Lösungsmittel gleichzeitig bei Raumtemperatur durch kontinuierlichen Sog wieder entfernt wird.

Der derart behandelte Filter wird nunmehr gründlich mit Ringerlösung gewaschen, vorsichtig getrocknet und in einen Filterhalter eingespannt und gassterilisiert. Danach wird es in ein verschließbares Gefäß mit Möglichkeiten zur Luft- und Kohlendioxidzuleitung bzw. zur kontinuierlichen Absaugung und Zuführung von Nährmedium derart gestellt, daß der erfindungsgemäß beschichtete Filter von Nährmedium auf beiden Seiten völlig bedeckt ist.

Nach einer ca. 24 Stunden dauernden Äquilibrierungsperiode wird der Filter aus dem Gefäß genommen und in bekannter Art und Weise mit Entodermzellen (aus einem 10 Tage alten Hühnerembryo) auf einer Seite beimpft und anschließend in dem nährmediumenthaltenden Gefäß unter den üblichen Bedingungen inkubiert.

Nach ca. drei bis vier Tagen hat sich ein gleichmäßiger Epithelzellrasen auf der beschichteten Filteroberfläche ausgebildet. Nun wird die Na-Ionen-Konzentration im Nährmedium auf der von den Zellen bewachsenen Filterseite kontinuierlich erhöht, wohingegen die Na-Ionen-Konzentration auf der anderen Seite im reziproken Verhältnis gesenkt wird: in der Regel wird alle acht Stunden eine Konzentrationserhöhung bzw. -senkung von 3 % des Ausgangswertes vorgenommen. Nachdem innerhalb von ca. drei Tagen manipulatorisch eine Konzentrationsdifferenz von ca. 40 bis 50 % zwischen den beiden Seiten errichtet wurde, behält man die erreichten Na-Konzentrationen bei bzw. erhöht nur noch die Na-Konzentration auf der zellbewachsenen Seite (für weitere 2 Tage, in gleichen Konzentrationsänderungsschritten).

0184640

Die aktive Stoffwechselleistung des Na-Ionentransports zeigt sich anschließend darin, daß dann bei konstanter Na-Ionenzufuhr auf beiden Seiten die Na-Ionenkonzentration auf der zellbewachsenen Filterseite unter die der anderen Seite absinkt und auch aufrechterhalten wird.

0184640

## Patentansprüche

1. Unterlage für das Zellwachstum in vitro, dadurch herstellbar, daß man synthetische oder natürliche Lipoide als Reinsubstanzen oder als mengenmäßig definierte Mischungen in einem unpolaren Lösungsmittel oder -gemisch löst oder suspendiert, die erhaltene Lösung oder Suspension mit einem Polyamid, Polypropylen, Polyalkylenterephthalat oder Fluorpolymerisat mit einer Durchlässigkeit von Molekülen mit einem Molekulargewicht von mindestens 400 000 Dalton oder einem Partikeldurchmesser von 500 nm, mechanischer Stabilität und Flexibilität und kurzzeitiger Beständigkeit gegen ein unpolares Lösungsmittel oder -gemisch in innigen Kontakt bringt und anschließend das Lösungsmittel oder -gemisch wieder vollständig entfernt.

2. Verwendung der Unterlage des Anspruchs 1 nach Ausbildung eines dichten Zellrasens auf ihrer Oberfläche zum induzierten, stoffwechselaktiven, vektoriellen Stofftransport.